Europäisches Patentamt

(19) European Patent Office

Office européen des brevets

(11) Publication number: **0 005 922**

A1

(12) **EUROPEAN PATENT APPLICATION**

(21) Application number: **79300807.9**

(22) Date of filing: **10.05.79**

(51) Int. Cl.²: **A 61 K 7/025**

(30) Priority: **19.05.78 GB 2083578**

(43) Date of publication of application:
**12.12.79 Bulletin 79/25**

(84) Designated Contracting States:
**AT BE CH DE FR GB IT LU NL SE**

(71) Applicant: **SMITH & NEPHEW COSMETICS LIMITED**
**Hook Rise South**
**Surbiton, Surrey(GB)**

(72) Inventor: **Julietti, Frederick John**
**9, Roxborough Avenue**
**Isleworth Middlesex(GB)**

(72) Inventor: **Bowers, Dale Daxon**
**15, Rythe Court**
**Thames Ditton Surrey(GB)**

(74) Representative: **Clifford, Frederick Alan et al,**
**MARKS & CLERK 57/60 Lincoln's Inn Fields**
**London W.C.2A 3LS(GB)**

(54) **Lip cosmetic.**

㊼ One or more waxes or wax-like substances is dissolved in a volatile solvent and a suitable pigment dispersed throughout the formulation to give a lip cosmetic paintable on as a liquid layer 7 and drying in e.g. 2 - 3 minutes to a solid fine-crystal layer 9 keyed into the skin rugosities 5 to improve its adherence.

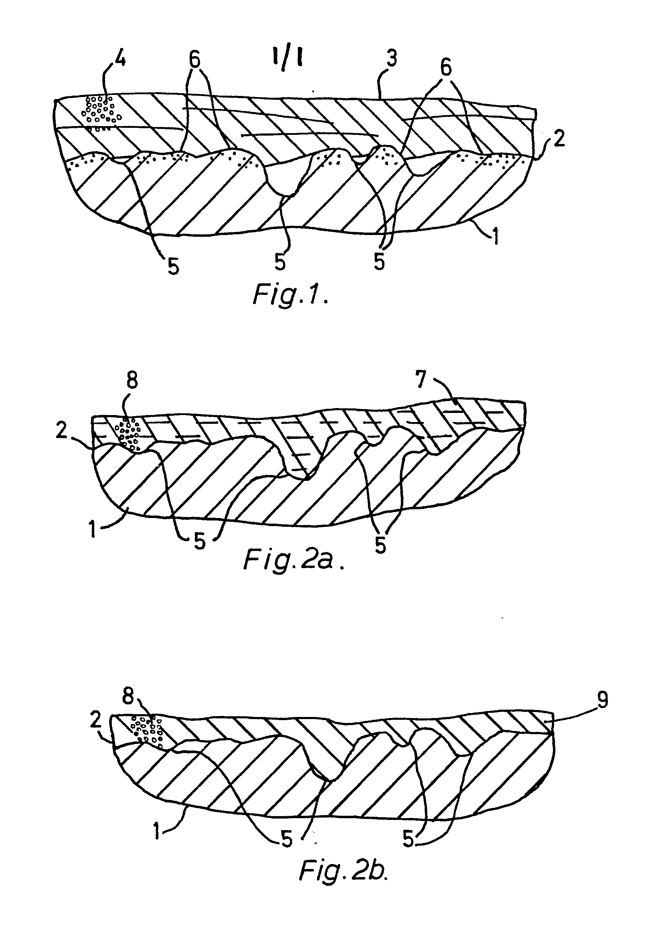

The wax can be inter alia carnauba, lanolin, bees-wax, paraffin or microcrystalline wax. The solvent can be odourless kerosene or dimethyl silicones. Preferably the solvent content, or a gelled or emulsified formulation is adjusted to keep the pigments in suspension (Figures 2a and 2b)

Fig.2a.

Fig.2b.

0005922

"LIP COSMETIC"

This invention relates to a new formulation of lip cosmetic.

Hitherto lip cosmetics have been made from waxes softened with suitable oils and coloured to varying degrees with pigments or iridescent powders.

Such formulations have been provided in a wide range of consistencies, ranging from a firm stick which is applied directly to the skin to a viscous liquid or paste which is applied by a brush or roller. In each case, however, the applied formulation has formed a layer upon the skin of essentially the same composition as that of the formulation in the stick or container. This soft waxy layer soon becomes displaced or removed by normal movements of the mouth or tongue, and thus needs frequent repair.

There have been proposals to supplement the action of such conventional lip cosmetics in stick or paste or liquid form by incorporating dyestuffs. The dyestuffs in question have usually been fluorescein derivatives, and penetrate into the top layers of skin to form a permanent coloured layer lasting several hours or days. Such formulations, however present their own problems since the dyestuffs are difficult to dissolve, giving rise to colour variation in use and special ingredients must be incorporated to overcome this. Moreover, such concentrations of dyestuffs are currently felt to be potentially harmful to the user, and recent legislation has drastically reduced the permissible numbers and concentrations of such materials in formulations because of a concern that they are potential irritants.

It is also known, within the general field of cosmetics, to produce an eye-shadow cosmetic in which the pigment and wax are associated with a volatile solvent. These formulations were developed after it was observed that mere softened and pigmented wax formulations, although capable of smooth application, tended to accumulate in the natural creases of the eye-lids after a period of wear. The use of volatile solvents for the wax and pigment allowed the application of a uniform layer which retained its uniformity over an acceptable period.

The present invention is based upon the surprising realisation that formulations resembling those in use for eye shadow can safely and effectively be used as lip cosmetic formulations.

Thus, the invention provides a lip cosmetic based upon an orally acceptable non-toxic wax at least partially dissolved in a readily volatile organic solvent.

With the criteria of the cosmetic industry, it is a surprising step to apply an eye

3.

0005922

cosmetic type of formulation to a lip cosmetic.
Firstly, there is a great difference in skin type
between the skin of the eye-lids, which at the time of
application is relatively soft and smooth, and the
skin of the lips, which varies greatly from person
to person but, whether dry or moist, is typically
somewhat ridged and puckered and of a different type
from the skin of the eye-lids. Secondly, there is a
great difference in mode of use. The typical wear
imparted to an eye shadow cosmetic is by the skin
of the eye-lids coming into contact when the eyes are
blinked, with no side-to-side abrasion and no
applied moisture. The continued effect upon the eye-
lids is to tend to cause an applied layer to migrate
towards the inside of the normal crease line. On the
other hand, the skin of the lips is much more exposed
to abrasion, with side-to-side or to and from movement
in talking or eating. Also, the tongue habitually
passes at least on extent over some of the lip surface,
and the environment can vary from very dry at the outer
edges of the lips to continually moist at the inner
edges. There is also in certain instances a creasing
problem at the corner of the lips,and in fact the
skin of the lips varies considerably from one person
to another. Thirdly, when applying eye cosmetic it
is not usually necessary to concern oneself with the
taste, texture, or oral toxicity of the materials;

0005922

lip cosmetics must bear all of these considerations in mind. It is also a feature of the present invention that the volatile solvent dries so quickly on the relatively warm skin of the lips as to present no problem of acceptability or toxicity of solvent leaving a layer of the wax material uniformly over the whole of the lip surface in such a way as to resist normal abrasion, and of a small crystal habit either because of rapid evaporation or because of the intentional inclusion of a microcrystalline wax, as discussed below, neither expedient being present or necessary with an eye cosmetic.

Although the applicants do not wish to be limited by any particular discussion as to the theory of their invention, it is believed that, with prior lip cosmetics, the coating was either a waxy material applied as a smooth layer over the skin irregularities or was a dyed surface layer extending into the upper skin cells. The coating of the present invention appears to be a layer of minute wax crystals deposited by rapid evaporation of the volatile solution and faithfully following the contours of the lips so as to provide a uniform layer adherent against normal abrasion.

The invention accordingly consists in a lip cosmetic comprising (a) at least one orally acceptable non-toxic wax or wax-like substance,

0005922

at least partially dissolved in (b) a solvent or solvent mixture sufficiently volatile to be evaporated by the heat of the lips, together with (c) one or more suitable pigments or like solid materials dispersed throughout the formulation .

By "orally acceptable" is meant that the wax has no untoward greasy feel to the tongue, that it should not be readily transferred to the mouth and that the unavoidable traces that are so transferred should not feel greasy or gritty or taste unpleasant, accumulate on the teeth or have any long-term adverse effects. The term is basically linked to unpleasant taste or feel, on the lips or in the mouth, and any given wax will either have known properties to a man skilled in the cosmetic art or have properties which can be established by simple and routine experiment.

By "non-toxic" is meant freedom not only from acute effects, but also from long term unsuitability again, to a man in the art toxicity considerations for cosmetic ingredient are a matter of common knowledge or of routine experiment.

A wax is a complex mixture of compounds usually solid and possessing a characteristic sheen at room temperature having the property of melting to a mobile liquid and resolidifying on cooling in the same composition in all significant respects. Predominately waxes are based on esters of higher mono-

hydric (more seldom di hydrics ) alcohols, containing in addition free acids, free alcohols and often free hydrocarbons.

The waxes which can be used (alone or in admixture can thus arise from natural (animal, vegetable or petroleum) sources or from special syntheses .

Among animal waxes, broadly so-called, are bees-wax and lanolin waxes. Bees-wax consists predominately of the palmitic ester of myricyl alcohol $CH_3(CH_2)_{14} COOC_{31}H_{63}$ containing in addition cerotic acid (10 - 14% by weight) and hydrocarbons (12-17% by weight). Lanolin waxes, or "wool wax", the neutral portion of wool fat has a somewhat different composition for it contains the complex alcohol cholesteral belonging to the carbocyclic compounds in addition to higher fatty acids and hydrocarbon.

Among vegetable waxes is carnauba wax, the wax attained from a Brazilian fan-palm. It consists of the myricyl ester of cerotic acid $C_{25}H_{51} COOC_{31}H_{63}$ together with the free acids (Carnauba acid, cerotic acid) higher alcohols and a hydrocarbon.

Petroleum, or paraffin, waxes generally arise from still residues in petroleum refinery. Their compositions vary widely, depending both on the feed stock and the refining conditions but generally speaking they are all hydrocarbons, predominantly straight chain and approximately of carbon number between $C_{16}$ and $C_{40}$.

Waxes can be made synthetically, tailored to a desired mixture of characteristics, by a process akin to the Fischer-Tropsch synthesis, again being mixtures as described above.

It should moreover be borne in mind that certain polymeric materials possess wax-like characteristics at lower molecular weight. This is especially true for polyolefins such as polyethylene which at lower molecular weights (e.g. 500 - 5000) possess a characteristic waxy sheen, a relatively sharp low melting point and a generally waxy nature, as would be expected from their general similarity to long-chain hydrocarbons, esters, etc. Such polymeric substances are referred to herein as "wax-like substances".

Cutting across the above classification, but predominantly found in the paraffin wax category or their synthetic analogues are the "microcrystalline" waxes. In these the crystals formed on solidification are significantly smaller than the crystals formed from the other waxes. The reasons for this are not fully elucidated but may be related to the presence of a higher proportion of branched-chain hydrocarbon residues in the components. In any event, they not only form small crystals themselves but also modify the crystal structure of other waxes when used in blends with them. The term "microcrystalline wax" is an art term, known to the cosmetic chemist and appearing as such in reference books and catalogues.

Further undue discussion of the chemical nature of these waxes would be misleading since no cosmetic chemist would choose a wax for this invention primarily on its chemical structure but on its recorded or easily determined toxicity, organoleptic properties, physical properties based on crystallinity, availability and, having regard to the particular nature of the invention, compatibility with solvent and ability to allow solvent to evaporate at a suitable rate.

It is preferred for the wax to be in admixture with a natural or synthetic resinous material material compatible both with the wax and with the underlying skin. Such a resin is provided to increase the resistance of the wax film to water and abrasion. The resins which are suitable are usually polymeric and can be of natural or synthetic origin provided they are non-toxic and do not detract from the performance of the wax/solvent combination. Examples are rosin and the aryl-sulphonamide resins.

Basically, any readily volatile organic solvent can be used which is acceptable without irritation to the lips. Typical solvents that come to mind are lower alcohols, ethers, or esters. However, preferred examples of solvents, for cheapness, are the aliphatic hydrocarbons especially kerosenes (e.g. odourless kerosenes)or volatile silicones (such as dimethyl silicones) since these solvents are readily driven off by the heat of the lips and thus can exert no toxic effect upon the user. Moreover, by a judicious combination of solvents, it is possible to select a boiling range which will give an acceptable drying time for any combination of waxes.

The pigments to be used can of course be those already well known in the art e.g. those based on carefully purified metal oxides or hydroxides, already well known in the cosmetic art such as red iron oxide and titanium dioxide. Other non-pigment solids e.g. iridescent powders such as titanium -coated mica can also be incorporated.

Since typically, volatile solvents are of rather lower viscosity than the oils previously used in solid wax formulations care should be taken to keep pigments in adequate suspension in the formulation.

One way of doing this is to control the proportion of solvent which in a broad preferred aspect ranges from 10 - 80% w/w of the product but which more preferably ranges from 20 - 50% w/w.

Another method of ensuring that the pigments remain in suspension throughout the formulation is to add a gelling agent. Among those which can be used in suitable formulations are cellulose gums, gelatin, montmorillonite clays and aluminium stearate, the criteria again being toxicity, organoleptic properties and formulation compatibility rather than chemical nature.

A third way of ensuring that the pigments remain in suspension is to provide the formulation, at least in part, as an emulsion. Thus, it is possible to emulsify water into the system, by use of suitable blends of suitable emulsifying agents (from such as:- sodium lauryl sulphate, ethanolamine stearate, diethanolamine stearate, a hydroxyalkyl ether, or glyceryl monolaurate) and thereby create a water-in-oil emulsion, which stabilises the distribution of pigment particles due to increased viscosity of the product and improved "wetting" of the pigment particles.

The overall proportions should be chosen so that the formulation dries on the lips in an acceptable time, which is typically 2 - 3 minutes, to fit in the time scale of other cosmetic applications.

The formulation according to the invention will be generally a viscous liquid or paste. It may be varied in detail so as to provide either a matte or glossy surface appearance after evaporation. Moreover, conventional additives such as perfume or

11.    0005922

or flavouring can be added to the formulation.

In that the formulation contains a volatile solvent it should be packaged in a suitable container with an effective closure. Also, to provide the neck of such container in such a size or configuration as to wipe a brush or foam pad withdrawn from the container, thereby to avoid excessive application and too long a drying time, would also be an advantage.

The invention will be further described with reference to the following specific examples.

| 1. Hydrocarbon Emulsion | wt. % |
|---|---|
| Long chain microcrystalline wax available as "Wax K 114" from Astor Chem. Co. | 10.0% |
| Oleyl ethoxylate emulsifier | 2.5% |
| Stearic Acid | 0.5% |
| Triethanolamine | 0.2% |
| Bentone gelled with Kerosene ($160^{o}$-$176^{o}$) | 10.1% |
| $160^{o}$-$176^{o}$ Kerosene fraction | 30.0% |
| Red Iron Oxide (Cosmetic grade) | 2.5% |
| Titanium Dioxide (Cosmetic grade) | 2.5% |
| Water | 40.0% |
| | 98.2% |

2. Hydrocarbon/Wax Blend

| | |
|---|---|
| Wax K114, as 1 | 10.0g |
| Polyethylene, low m  w | 0.2g |
| Red Iron Oxide, as 1 | 2.5g |
| Titanium dioxide | 2.5g |
| Titanium-coated mica (iridescent powder) | 5.0g |
| $160^{o}$ – $176^{o}$C Kerosene fraction | 30.0g |

3. Silicone Wax Blend

| | |
|---|---|
| Wax, as 1 | 10.0g |
| Red Iron Oxide, as 1 | 2.5g |
| Titanium Dioxide, as 1 | 2.5g |
| Titanium coated mica, as 2 | 5.0g |
| Dimethyl silicone resin | 30.0g |

Each of the above formulations gave a stable pasty product which could be applied to the skin of the lips by brush or foam pad and dried within 3 minutes to a layer uniformly following the contour and roughness of the lips of a microcrystalline nature and good adherence and resistance to abrasion.

The invention will be further described with reference to the accompanying drawings, in which:

Figure 1 is a diagram showing how a conventional lipstick is believed to be located on the skin of the lips,

Figure 2a is a like diagram showing the lip cosmetic of the invention immediately after application,

Figure 2b is a like diagram to that of Figure 2a showing the lip cosmetic of the invention after drying.

Figure 1 shows a portion of lip-tissue 1 with a soft but somewhat uneven surface at 2 over which is applied by repeated smears or streaks a wax layer 3 containing pigment dispersed throughout as at 4. This prior art arrangement means that the applied lipstick does not reach into, and hence does not key into the rugosities 5 on the surface. Also, any included dyestuff, incorporated to give the relatively unadherent wax layer some apparent wear resistance only dyes the underlying skin at intervals e.g. at 6.

Figure 2 shows a liquid or pasty layer of formulation 7 of the invention, with suspended pigment as at 8, covering and filling all rugosites 5.

Figure 2b shows Figure 2a two minutes later, after solvent evaporation. A uniform finely crystalline, unsmeared layer 9 keyed into rugosities 5 covers the whole surface.

**0005922**

CLAIMS :

1.      A lip cosmetic comprising (a) at least one
orally acceptable non-toxic wax or wax-like substance,
at least partially dissolved in (b) a solvent or solvent
mixture sufficiently volatile to be evaporated by the
heat of the lips, together with (c) one or more
suitable pigment or like solid materials dispersed
throughout the formulation.

2.      A lip cosmetic as claimed in claim 1 in
which the wax or wax-like substance is one of, or a
mixture of two or more of bees-wax, lanolin wax,
carnauba wax, paraffin wax or a low molecular polyolefin
wax-like material.

3.      A lip  cosmetic as claimed in claim 1, or
2 in which the wax or wax-like substance contains
a microcrystalline wax.

4.      A lip cosmetic as claimed in claim 1, 2 or
3 in which the wax is admixed with a natural or
synthetic resinous material to increase the resistance
of the wax film to water and abrasion.

5.      A lip cosmetic as claimed in any of claims
1 to 4 wherein the solvent is a kerosene or a dimethyl
silicone.

6.    A lip cosmetic as claimed in any one preceding claim wherein the solvent is present in an amount from 10 - 80% w/w in the formulation so as to at least assist in keeping the pigments in suspension.

7.    A lip cosmetic as claimed in claim 6 in which the solvent proportion is 20 - 50% w/w.

8.    A lip cosmetic as claimed in any one preceding claim comprising a gelling agent so as to at least assist in keeping the pigments in suspension.

9.    A lip cosmetic as claimed in claim 8 in which the gelling agent includes cellulose gums, gelatin, montmorillonite, clays, or aluminium stearate.

10.    A lip cosmetic as claimed in any one preceding claim comprising at least in part a water-in-oil emulsion so as to at least assist in keeping the pigments in suspension.

11.    A lip cosmetic as claimed in claim 10 in which the emulsifying agent is one of, or a blend chosen from sodium lauryl sulphate, ethanolamine stearate, diethanolamine stearate, a hydroxyalkyl ether or glyceryl monolaurate.

12.    A lip cosmetic as claimed in any one preceding claim which dries on the lips in 2 to 3 minutes.

13.    A lip cosmetic as claimed in any one preceding claim in the form of a viscous liquid.

14.    A lip cosmetic as claimed in any one of claims 1 to 13 in the form of a paste.

*Fig.1.*

*Fig.2a.*

*Fig.2b.*

0005922

# EUROPEAN SEARCH REPORT

**European Patent Office**

Application number

EP 79 30 0807

| | DOCUMENTS CONSIDERED TO BE RELEVANT | | CLASSIFICATION OF THE APPLICATION (Int. Cl.²) |
|---|---|---|---|
| Category | Citation of document with indication, where appropriate, of relevant passages | Relevant to claim | |
| X | US - A - 3 088 876 (BUTH)<br>* Column 1, line 52 - column 4, line 13; the examples; the claims * | 1-14 | A 61 K 7/025 |
| | -- | | |
| X | FR - A - 915 416 (CELORON DE BLAIN-VILLE)<br>* Entirely * | 1-14 | |
| | -- | | |
| A | FR - A - 935 150 (MARTIN-GORDON) | 1 | TECHNICAL FIELDS SEARCHED (Int.Cl.²) |
| A | FR - A - 998 013 (SOC. LES PROD. DE BEAUTE YAMIL) | 1 | A 61 K 7/025<br>A 61 K 7/027 |
| A | GB - A - 525 337 (KILMIST) | 1 | |
| A | LU - A - 52 961 (VOISIN) | 1 | |
| A | CH - A - 224 313 GIVAUDAN) | 1 | |
| A | CH - A - 264 133 (CZAKO) | 1 | |
| A | CH - A - 519 913 (VOISIN) | 1 | |
| | ---- | | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 13-08-1979 | JONAS |

EPO Form 1503.1   06.79